# EUROPÄISCHE PATENTSCHRIFT

(11) **EP 3 621 944 B1**
(45) Veröffentlichungstag und Bekanntmachung des Hinweises auf die Patenterteilung: **08.10.2025**
(21) Anmeldenummer: 17805184.3
(22) Anmeldetag: 27.11.2017
(51) Int. Cl.: C07C 209/90, C07C 211/36

(54) **VERFAHREN ZUR STABILISIERUNG VON ZUMINDEST MONOALKYLSUBSTITUIERTEN DIAMINOCYCLOHEXANEN**
METHOD FOR THE STABILISATION OF AT LEAST MONO-ALKYL-SUBSTITUTED DIAMINOCYCLOHEXANES
PROCÉDÉ DE STABILISATION DE DIAMINOCYCLOHEXANES AU MOINS MONOALKYL-SUBSTITUÉS

(30) Priorität: 29.11.2016 EP 16201152
(43) Veröffentlichungstag der Anmeldung: 18.03.2020
(73) Patentinhaber: BASF SE, 67056 Ludwigshafen (DE)
(72) Erfinder: JAEGLI, Stephanie, 67056 Ludwigshafen (DE); ERNST, Martin, 67056 Ludwigshafen (DE); PANCHENKO, Alexander, 67056 Ludwigshafen (DE); HETTCHE, Frank, 67056 Ludwigshafen (DE); LUDOLPH, Bjoern, 67056 Ludwigshafen (DE)
(74) Vertreter: BASF IP Association
(86) Internationale Anmeldenummer: PCT/EP2017/080558
(87) Internationale Veröffentlichungsnummer: WO 2018/099871

(56) Entgegenhaltungen:
- US-A- 3 922 306
- US-A- 5 516 935
- MICHAEL ASH ET AL: "Industrial Chemical Thesaurus", 1 June 2009 (2009-06-01), Endicott, pages 56 - 57, XP055448549, ISBN: 978-1-934764-06-0, Retrieved from the Internet <URL:https://dytek.invista.com/~/media/INVISTA/Intermediates/DYTEK/Documents/brochures/Color-Stabilized%20DYTEK%20DCH-99%20amine%20%20Adducts%20for%20Epoxy%20Curing%20pdf.ashx>

## Beschreibung

Die vorliegende Erfindung betrifft ein Verfahren zur Stabilisierung von zumindest monoalkylsubstituierten Diaminocyclohexanen, in dem mindestens ein Reduktionsmittel (R) und gegebenenfalls Wasser zu einer Zusammensetzung (ZE) zugegeben werden. Die Zusammensetzung (ZE) enthält mindestens ein zumindest monoalkylsubstituiertes Diaminocyclohexan (A) und gegebenenfalls Wasser. Durch die Zugabe des mindestens einen Reduktionsmittels (R) und gegebenenfalls Wasser zu der Zusammensetzung (ZE) wird eine Zusammensetzung (ZP) erhalten, die mindestens 0,05 Gew.-% Wasser aufweist, bezogen auf das Gesamtgewicht der Zusammensetzung (ZP). Das mindestens eine zumindest monoalkylsubstituierte Diaminocyclohexan (A) ist aus einer der Verbindungen gemäß den allgemeinen Formeln (I), (II) oder (III) ausgewählt, in denen R₁, R₁', R₂, R₂', R₃, R₃', R₄ und R₄' unabhängig voneinander ausgewählt sind aus H und C₁-C₄-Alkyl, wobei mindestens ein Rest R₁, R₁', R₂, R₂', R₃, R₃', R₄ oder R₄' C₁-C₄-Alkyl ist, und wobei zunächst eine Destillation der Zusammensetzung (ZE) unter Abtrennung höher siedender Nebenprodukte und unter Erhalt einer destillierten Zusammensetzung (DZE), die das mindestens eine zumindest monoalkylsubstituierte Diaminocyclohexan (A) und gegebenenfalls Wasser enthält, durchgeführt wird, worauf die Zugabe des mindestens einen Reduktionsmittels (R) und gegebenenfalls Wasser zu der destillierten Zusammensetzung (DZE) unter Erhalt der Zusammensetzung (ZP) erfolgt, wobei die Zusammensetzung (ZP) 0,05 bis 3 Gew.-% Wasser enthält, bezogen auf das Gesamtgewicht der Zusammensetzung (ZP). Weitere Gegenstände der Erfindung sind die Zusammensetzung (ZP) sowie deren Verwendung beispielsweise zur Herstellung von Härtern für Epoxyharze.

Cycloaliphatische Diamine wie alkylsubstituierte Diaminocyclohexane haben im Vergleich zu entsprechenden aromatischen Diaminen in der Regel eine bessere Licht-, UV- und Witterungsbeständigkeit, weshalb sie für verschiedene Produkte, wie beispielsweise Beschichtungsmaterialien, wichtige Ausgangsverbindungen darstellen. Viele cycloaliphatische Amine sind in Reinform üblicherweise farblose Flüssigkeiten, die allerdings aufgrund von Verunreinigungen, welche beispielsweise von Metallen oder Metallverbindungen oder von Verunreinigungen an Nebenprodukten aus dem Herstellungsverfahren stammen, oft dazu neigen, sich im Laufe der Zeit zu verfärben. Durch die Tendenz zur Verfärbung ist der Einsatzbereich von cycloaliphatischen Aminen eingeschränkt, weshalb es wünschenswert ist, solche Materialien entsprechend aufzureinigen und die Verfärbung über einen möglichst langen Zeitraum zu verhindern oder möglichst gering zu halten. Da die Bildung von farbgebenden Verunreinigungen in jedem Herstellungsverfahren für Amine und in jedem Aminprodukt unterschiedlich ausfällt, ist die Entfärbung sowie Farbstabilisierung von manchen Aminen nicht automatisch auch für andere Amine geeignet.

Im Stand der Technik werden daher teilweise sehr unterschiedliche Herangehensweisen offenbart, um Amine kurzfristig zu entfärben oder langfristig deren Farbstabilität zu verbessern:
So offenbart beispielsweise US 3,922,306 ein Verfahren zur Entfärbung von aliphatischen Aminen, in dem ein aliphatisches Amin und ein Alkaliborhydrid für mehrere Stunden auf 50 bis 70 °C erhitzt und anschließend voneinander getrennt werden. Gemäß US 3,922,306 konnte eine entsprechende Entfärbung nicht mittels anderer Reduktionsmittel wie Natriumsulfit, Natriumdithionit, Hydrazin oder Phosphinsäure erreicht werden.

In US 7,169,268 wird ein Verfahren zur Herstellung eines farbstabilen tertiären Amins offenbart, in dem ein tertiäres Amin in Gegenwart von Ethylendiamin oder eines Ethylenaminderivats destilliert wird. Das destillierte tertiäre Amin verfärbt sich weniger schnell als entsprechendes unbehandeltes tertiäres Amin, allerdings zeigen die Farbstabilitätsuntersuchungen in US 7,169,268, dass auch nach kurzer Zeit an Luft die Verfärbung des Amins zunimmt. Eine längerfristige Farbstabilität kann dadurch erreicht werden, dass das tertiäre Amin in einer Inertgasatmosphäre gelagert wird.

US 4,731,165 offenbart ein Verfahren zur Entfärbung von Triethylentetraamin, gemäß dem Triethylentetraamin mit einem Ionentauscherharz auf Basis von Sulfonsäure katalytisch aufgereinigt wird. Die Aufreinigung des Triethylentetraamins mit dem Ionentauscherharz erfolgt unter reduziertem Druck und erhöhten Temperaturen und im Anschluss an den Aufreinigungsschritt wird das Ionentauscherharz von Triethylentetraamin mittels Destillation abgetrennt. Das Verfahren ist gemäß US 4,731,165 auch zur Aufreinigung anderer Polyalkylenpolyamine geeignet, enthält allerdings keine Angaben bezüglich der längerfristigen Farbstabilität der Amine.

US 5,362,914 beschreibt ein kontinuierliches Verfahren zur Verringerung der Verfärbung von Polyethylenpolyaminen, bei dem Polyethylenpolyamine in Gegenwart eines Cobalt-, Kupfer- und Chrom-haltigen Katalysators in einer Wasserstoffatmosphäre bei erhöhten Temperaturen und erhöhtem Druck hydriert werden. Die Polyethylenpolyamine können vor der Hydrierung destilliert werden oder als Rohprodukt eingesetzt werden und im Anschluss an die Hydrierung erfolgt ein weiterer Destillationsschritt. Das in US 5,362,914 beschriebene Verfahren ist zwar zur Entfärbung von Polyethylenpolyaminen geeignet, allerdings neigen auch die so erhaltenen Polyethylenpolyamine an Luft zu Verfärbung und sollten gemäß US 5,362,914 unter Stickstoffatmosphäre gelagert werden.

Ein alternatives Verfahren zur Entfärbung von Polyethylenpolyaminen wird in US 4,609,436 offenbart, in dem Polyethylenpolyamine mit einem chlorhaltigen Kohlenwasserstoff versetzt und bei erhöhten Temperaturen gerührt werden. Im Anschluss an die Behandlung der Polyethylenpolyamine mit dem chlorhaltigen Kohlenwasserstoff erfolgt eine Destillation, in der das Polyethylenpolyamin vom chlorhaltigen Kohlenwasserstoff abgetrennt wird.

US 6,774,264 offenbart ein Verfahren zur Verbesserung der Farbstabilität von N,N-Dialkylalkanolaminen durch Hydrierung von entsprechenden Aminen in Gegenwart eines Palladiumkatalysators unter wasserfreien Bedingungen. Die Hydrierung erfolgt in US 6,774,264 vorwiegend zur Entfernung von ungesättigten Nebenprodukten, die während der Herstellung von N,N-Dialkylalkanolaminen entstanden sind.

In US 5,847,221 wird ein Verfahren zur Entfärbung von Alkanolaminen oder Alkylenaminen offenbart, bei dem das entsprechende Amin mit einem polymeren, festen Säurekatalysator in Gegenwart von geringen Mengen an Wasser bei erhöhten Temperaturen und erhöhtem Druck für mehrere Stunden behandelt wird, um Metallkatalysatorreste aus dem jeweiligen Herstellungsverfahren der Amine und Verbindungen mit konjugierten Doppelbindungen zu entfernen oder zu zersetzen. Im Anschluss an den Entfärbungsschritt kann ein Destillationsschritt erfolgen.

US 5,359,139 offenbart ein Verfahren zur Behandlung von tertiären Aminen, in dem ein tertiäres Amin mit Ascorbinsäure versetzt wird, die anschließend destillativ vom tertiären Amin wieder abgetrennt wird. In den so behandelten tertiären Aminen kann dadurch eine Verfärbung der tertiären Amine unter sauren Bedingungen weitestgehend vermieden werden.

Während die vorstehend genannten Verfahren Möglichkeiten zur sofortigen Aufreinigung verschiedener Amine beschreiben, sind Zusammensetzungen mit langfristig farbstabilen Aminen in der Regel durch den Einsatz bestimmter Stabilisatoren zugänglich.

So offenbart beispielsweise US 4,602,108 die Farbstabilisierung von linearen oder verzweigten aliphatischen Aminen durch geeignete Stabilisatoren wie Nitrilotrismethylenphosphonsäure, 8-Hydroxychinolin oder Ethylendiamintetraessigsäure als Stabilisator. Der Stabilisator ist dabei nur wenig bis schlecht löslich im aliphatischen Amin.

WO 2011/084865 offenbart eine Zusammensetzung, welche ein oxidationsempfindliches Amin sowie einen Oxidationsinhibitor enthält. Der Oxidationsinhibitor kann dabei ein Radikalfänger wie beispielsweise Phenylimidazol oder Glutamin oder ein Antioxidant wie Ascorbinsäure sein. Der Einsatz des Oxidationsinhibitors soll vor allem die Bildung von Formaldehyd und Dimethylformamid verhindern, welche bei den in WO 2011/084865 beschriebenen, oxidationsempfindlichen Aminen während der Lagerung an Luft oft gebildet werden.

In *"*Color-Stabilized DYTEK® DCH-99 amine Adducts for Epoxy Curing, Invista, 14. März 2012" wird die Stabilisierung von 1,2-Diaminocyclohexan offenbart, welches ein Stabilisatorsystem aus Natriumborhydrid, Wasser, Benzylalkohol, Triethylamin sowie einem Epoxyharz enthält, um die Verfärbung des Amins während der Lagerung an Luft zu vermindern. Zusätzliche Mengen an Natriumborhydrid und Wasser erhöhen die Farbstabilität. Der Gewichtsanteil des Stabilisatorsystems ist dabei deutlich größer als der Gewichtsanteil des 1,2-Diaminocyclohexans.

US 5,516,935 A offenbart ein Verfahren zur Herstellung von Diisocyanaten, bei dem ein aliphatisches oder cycloaliphatisches Amin mit Phosgen und einem Lösungsmittel in der Gasphase in einem Reaktor umgesetzt und anschließend aufgereinigt wird. Als cycloaliphatische Amine können beispielsweise cycloaliphatische Diamine wie 2,3-, 2,4 oder 2,6-Diamino-1-methylcyclohexane eingesetzt werden.

In Michael Ash et al., "Industrial Chemical Thesaurus", 1. Juni 2009, Seiten 56 bis 57, XP55448549 werden farbstabilisierte Aminaddukte offenbart, die durch Natriumborhydrid (NaBH₄) in Wasser stabilisiert werden. Die beschriebenen Aminaddukte werden durch Umsetzung von 1,2-Diaminocyclohexan mit einem Epoxyharz in Gegenwart einer Mischung aus Triethanolamin und Benzylalkohol als Reaktionsbeschleuniger erhalten.

Aufgabe der vorliegenden Erfindung ist die Bereitstellung eines Verfahrens zur Stabilisierung von zumindest monoalkylsubstituierten Diaminocyclohexanen.

Diese Aufgabe wird gelöst durch ein Verfahren zur Stabilisierung von zumindest monoalkylsubstituierten Diaminocyclohexanen, umfassend die Zugabe mindestens eines Reduktionsmittels (R) sowie gegebenenfalls Wasser zu einer Zusammensetzung (ZE), welche mindestens ein zumindest monoalkylsubstituiertes Diaminocyclohexan (A) sowie gegebenenfalls Wasser enthält, unter Erhalt einer Zusammensetzung (ZP), wobei die Zusammensetzung (ZP) das mindestens eine Reduktionsmittel (R), das mindestens eine zumindest monoalkylsubstituierte Diaminocyclohexan (A) und zusätzlich mindestens 0,05 Gew.-% Wasser aufweist, bezogen auf das Gesamtgewicht der Zusammensetzung (ZP), dadurch gekennzeichnet, dass das mindestens eine zumindest monoalkylsubstituierte Diaminocyclohexan (A) aus einer der Verbindungen gemäß den allgemeinen Formeln (I), (II) oder (III) ausgewählt ist 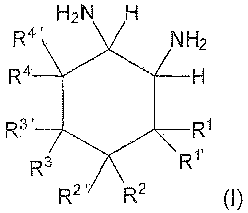 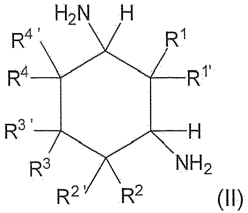 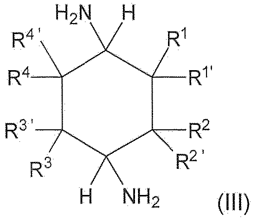 in denen
R₁, R₁', R₂, R₂', R₃, R₃', R₄ und R₄' unabhängig voneinander ausgewählt sind aus H und C₁-C₄-Alkyl, wobei mindestens ein Rest R₁, R₁', R₂, R₂', R₃, R₃', R₄ oder R₄' C₁-C₄-Alkyl ist,;
dadurch gekennzeichnet, dass zunächst eine Destillation der Zusammensetzung (ZE) unter Abtrennung höher siedender Nebenprodukte und unter Erhalt einer destillierten Zusammensetzung (DZE), die das mindestens eine zumindest monoalkylsubstituierte Diaminocyclohexan (A) und gegebenenfalls Wasser enthält, durchgeführt wird, worauf die Zugabe des mindestens einen Reduktionsmittels (R) und gegebenenfalls Wasser zu der destillierten Zusammensetzung (DZE) unter Erhalt der Zusammensetzung (ZP) erfolgt, wobei die Zusammensetzung (ZP) 0,05 bis 3 Gew.-% Wasser enthält, bezogen auf das Gesamtgewicht der Zusammensetzung (ZP).

Es wurde überraschenderweise festgestellt, dass die Verfärbung von zumindest monoalkylsubstituierten Diaminocyclohexanen bei der Lagerung an Luft deutlich verringert werden kann, wenn vor deren Lagerung mindestens ein Reduktionsmittel (R) sowie gegebenenfalls Wasser zugegeben wird.

Sowohl unter thermischer Belastung als auch während einer längeren Lagerung der stabilisierten zumindest monoalkylsubstituierten Diaminocyclohexane an Luft entstehen deutlich geringfügigere Farbvertiefungen als bei unstabilisierten, zumindest monoalkylsubstituierten Diaminocyclohexanen.

Ohne an eine Theorie gebunden zu sein, kann das mindestens eine Reduktionsmittel (R) die Bildung von Kondensationsprodukten der zumindest monoalkylsubstituierten Diaminocyclohexane oder gegebenenfalls anderer in der Zusammensetzung (ZE) enthaltener Amine sowie deren farbgebender Folgeprodukte verhindern, welche durch Lagerung von zumindest monoalkylsubstituierten Diaminocyclohexanen an Luft im Laufe der Zeit entstehen können.

Zudem erfordert das erfindungsgemäße Verfahren nicht die Entfernung von Wasser, das als Rückstand aus den entsprechenden Herstellungsverfahren in den zumindest monoalkylsubstituierten Diaminocyclohexanen enthalten sein kann, was aufwändige Trennverfahren zur Entfernung von Wasser aus den zumindest monoalkylsubstituierten Diaminocyclohexanen nicht erforderlich macht.

Im Rahmen der vorliegenden Erfindung bedeuten die Begriffe "(Farb-)Stabilität", "(Farb-)Stabilisierung" oder "(farb-)stabil", dass die Farbzahl eines Amins oder einer aminhaltigen Zusammensetzung über einen längeren Zeitraum unverändert niedrig bleibt oder vergleichsweise gering ansteigt. Die Bestimmung der Farbzahl erfolgt durch Langzeitlagertests, bei denen die Farbqualität einer Verbindung durch Messung der Transmission des eingestrahlten Lichts ermittelt wird. Hierzu wird eine Lösung einer bestimmten Konzentration in einer Küvette mit bekannter Schichtdicke mit einem Lichtstrahl einer definierten Wellenlänge durchstrahlt. Der Prozentsatz der durchgelassenen Lichtenergie ergibt bei gegebener Wellenlänge eine definierte Farbzahl. Gebräuchlich für schwach gefärbte Lösungen ist die Bestimmung der Hazen-Farbzahl nach der Platin-Cobalt-Farbskala nach APHA. Die Ermittlung der Hazen-Farbzahl nach der Platin-Cobalt-Farbskala nach APHA erfolgt in der Regel gemäß DIN EN ISO 6271.

Im Rahmen der vorliegenden Erfindung umfasst der Begriff "Kondensationsprodukt" alle Verbindungen, die in einer Reaktion unter Abspaltung von Wasser, Ammoniak, Kohlenstoffdioxid, Halogenwasserstoffen oder Alkoholen entstehen.

Die vorliegende Erfindung wird nachfolgend im Detail erläutert.

Die vorliegende Erfindung betrifft ein Verfahren zur Stabilisierung von zumindest monoalkylsubstituierten Diaminocyclohexanen.

Im erfindungsgemäßen Verfahren wird mindestens ein Reduktionsmittel (R) einer Zusammensetzung (ZE) zugegeben, welche mindestens ein zumindest monoalkylsubstituiertes Diaminocyclohexan als Komponente (A) enthält.

Die Zusammensetzung (ZE) ist das Gemisch, das das mindestens eine zumindest monoalkylsubstituierte Diaminocyclohexan (A) enthält. Das mindestens eine zumindest monoalkylsubstituierte Diaminocyclohexan (A) kann in beliebigen Mengen in der Zusammensetzung (ZE) enthalten sein. Sämtliche der nachfolgenden Angaben bezüglich der Zusammensetzung (ZE) beziehen sich daher auf das entsprechende Gemisch vor der Zugabe des mindestens einen Reduktionsmittels (R).

Die Zusammensetzung (ZE) enthält vorzugsweise mindestens 69 Gew.-%, bevorzugt mindestens 90 Gew.-% und besonders bevorzugt mindestens 94 Gew.-% des mindestens einen zumindest monoalkylsubstituierten Diaminocyclohexans (A), bezogen auf das Gesamtgewicht der Zusammensetzung (ZE).

In einer weiteren Ausführungsform enthält die Zusammensetzung (ZE) vorzugsweise mindestens 85 Gew.-%, bevorzugt mindestens 90 Gew.-% und besonders bevorzugt mindestens 95 Gew.-% des mindestens einen zumindest monoalkylsubstituierten Diaminocyclohexans (A), bezogen auf das Gesamtgewicht der Zusammensetzung (ZE).

Im Rahmen der vorliegenden Erfindung wird unter dem mindestens einen zumindest monoalkylsubstituierten Diaminocyclohexan (A) eine Verbindung verstanden, die einen Cyclohexanring mit zwei Aminogruppen sowie mit mindestens einem oder mehreren Alkylsubstituenten aufweist.

Alkylsubstituierte Diaminocyclohexane sind dem Fachmann prinzipiell bekannt und können durch alle dem Fachmann bekannten Verfahren hergestellt werden. Geeignete Verfahren zur Herstellung von alkylsubstituierten Diaminocyclohexanen umfassen beispielsweise die metallkatalysierte Hydrierung von alkylsubstituierten Diaminobenzolderivaten, wie sie beispielsweise in WO 2009/090179 und WO 2009/153123 offenbart wird.

Bevorzugt ist das mindestens eine zumindest monoalkylsubstituierte Diaminocyclohexan (A) mindestens ein zumindest monoalkylsubstituiertes 1,2-, 1,3- oder 1,4-Diaminocyclohexan, insbesondere mindestens ein zumindest monoalkylsubstituiertes 1,3-Diaminocyclohexan.

Bevorzugt ist das mindestens eine zumindest monoalkylsubstituierte Diaminocyclohexan (A) aus einer der Verbindungen gemäß den allgemeinen Formeln (I), (II) oder (III) ausgewählt 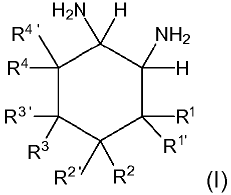 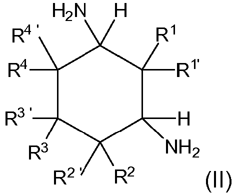 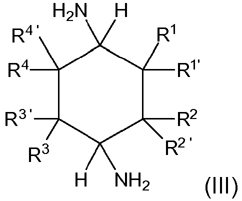 in denen
R₁, R₁', R₂, R₂', R₃, R₃', R₄ und R₄' unabhängig voneinander ausgewählt sind aus H und C₁-C₄-Alkyl, wobei mindestens ein Rest R₁, R₁', R₂, R₂', R₃, R₃', R₄ oder R₄' C₁-C₄-Alkyl ist.

Im Rahmen der vorliegenden Erfindung bedeutet die Bezeichnung C₁-C₄-Alkyl, wie sie beispielsweise für den Rest R¹ in den allgemeinen Formeln (I), (II) und (III) verwendet wird, dass dieser Substituent ein Alkylrest mit 1 bis 4 Kohlenstoffatomen ist. Der Alkylrest kann sowohl linear als auch verzweigt sein. Beispiele für Alkylreste sind Methyl, Ethyl, n-Propyl, n-Butyl sowie deren verzweigte Isomere.

Bevorzugt sind R₁, R₁', R₂, R₂', R₃, R₃', R₄ und R₄' unabhängig voneinander ausgewählt aus H oder Methyl.

Bevorzugt weist das mindestens eine zumindest monoalkylsubstituierte Diaminocyclohexan (A) die allgemeine Formel (I) auf, wobei R₁, R₁', R₂, R₂', R₃, R₃', R₄ und R₄' unabhängig voneinander ausgewählt sind aus H oder C₁-C₄-Alkyl, wobei genau ein Rest R₁, R₁', R₂, R₂', R₃, R₃', R₄ oder R₄' C₁-C₄-Alkyl ist.

Besonders bevorzugt ist das mindestens eine zumindest monoalkylsubstituierte Diaminocyclohexan (A) ausgewählt aus 1,3-Diamino-4-methylcyclohexan oder 1,3-Diamino-2-methylcyclohexan.

Das mindestens eine zumindest monoalkylsubstituierte Diaminocyclohexan (A) kann genau ein zumindest monoalkylsubstituiertes Diaminocyclohexan als auch Mischungen von zwei oder mehreren unterschiedlichen zumindest monoalkylsubstituierten Diaminocyclohexanen sein.

In einer bevorzugten Ausführungsform ist das mindestens eine zumindest monoalkylsubstituierte Diaminocyclohexan (A) eine Mischung aus 1,3-Diamino-4-methylcyclohexan und 1,3-Diamino-2-methylcyclohexan. Die Gewichtsanteile von 1,3-Diamino-4-methylcyclohexan und 1,3-Diamino-2-methylcyclohexan in dieser Mischung können dabei prinzipiell beliebig sein. Bevorzugt enthält das mindestens eine zumindest monoalkylsubstituierte Diaminocyclohexan (A) in dieser bevorzugten Ausführungsform 50 bis 95 Gew.-% an 1,3-Diamino-4-methylcyclohexan und 5 bis 50 Gew.-% an 1,3-Diamino-2-methylcyclohexan, bezogen auf das Gesamtgewicht des mindestens einen zumindest monoalkylsubstituierten Diaminocyclohexans (A).

In einer weiteren bevorzugten Ausführungsform ist das mindestens eine zumindest monoalkylsubstituierte Diaminocyclohexan (A) ausgewählt aus 1,3-Diamino-2-methylcyclohexan oder 1,3-Diamino-4-methylcyclohexan.

Darüber hinaus kann die Zusammensetzung (ZE) gegebenenfalls Wasser enthalten. Prinzipiell kann das Wasser auf beliebige Weise in die Zusammensetzung (ZE) zugeführt worden sein. Das Wasser kann beispielsweise ein Rückstand aus dem Herstellungsverfahren des mindestens einen zumindest monoalkylsubstituierten Diaminocyclohexans (A) sein.

Vorzugsweise weist die Zusammensetzung (ZE) einen Wassergehalt von mindestens 0,05 Gew.-%, bevorzugt von mindestens 0,08 Gew.-% und besonders bevorzugt von mindestens 0,1 Gew.-% auf, bezogen auf das Gesamtgewicht der Zusammensetzung (ZE).

Des Weiteren weist die Zusammensetzung (ZE) vorzugsweise einen Wassergehalt von höchstens 1 Gew.-%, bevorzugt von höchstens 0,8 Gew.-% und besonders bevorzugt von höchstens 0,5 Gew.-% auf, bezogen auf das Gesamtgewicht der Zusammensetzung (ZE).

In einer bevorzugten Ausführungsform weist die Zusammensetzung (ZE) einen Wassergehalt von 0,05 bis 1 Gew.-%, bevorzugt von 0,08 bis 0,8 Gew.-% und besonders bevorzugt von 0,1 bis 0,5 Gew.-% auf, bezogen auf das Gesamtgewicht der Zusammensetzung (ZE).

Die Zusammensetzung (ZE) kann neben dem mindestens einen zumindest monoalkylsubstituierten Diaminocyclohexan (A) sowie gegebenenfalls Wasser weitere Verbindungen enthalten.

Die weiteren Verbindungen sind vorzugsweise Rückstände aus dem jeweiligen Herstellungsverfahren des entsprechenden mindestens einen zumindest monoalkylsubstituierten Diaminocyclohexans (A). Diese Rückstände umfassen beispielsweise unsubstituierte oder zumindest monoalkylsubstituierte Aminocyclohexane, Verunreinigungen durch Metalle und Metallverbindungen von Hydrierungskatalysatoren, höhersiedende Nebenprodukte oder Lösungsmittelreste wie beispielsweise von Isopropanol, Isobutanol, tert-Butanol, Dimethoxyethan, Dioxan oder Tetrahydrofuran.

Als höher siedende Nebenprodukte werden solche Bestandteile bezeichnet, die einen höheren Siedepunkt aufweisen als das mindestens eine zumindest monoalkylsubstituierte Diaminocyclohexan (A), wobei der Siedepunkt der höher siedenden Nebenprodukte vorzugsweise um mindestens 2 °C, besonders bevorzugt um mindestens 4 °C und ganz besonders bevorzugt um mindestens 6 °C höher ist als der Normalsiedepunkt des mindestens einen zumindest monoalkylsubstituierten Diaminocyclohexans (A). Unter der Bezeichnung "Normalsiedepunkt" wird im Rahmen der vorliegenden Erfindung der Siedepunkt bei Normaldruck von 1,013 bar verstanden.

Sofern zwei oder mehrere zumindest monoalkylsubstituierte Diaminocyclohexane (A) in der Zusammensetzung (ZE) vorliegen, ist der Siedepunkt jedes höhersiedenden Nebenprodukts höher als der höchste Siedepunkt der zwei oder mehr zumindest monoalkylsubstituierten Diaminocyclohexane (A).

Die höher siedenden Nebenprodukte weisen vorzugsweise ein Molekülgewicht im Bereich von 100 bis 500 g/mol, mehr bevorzugt 120 bis 370 g/mol und besonders bevorzugt 150 bis 300 g/mol auf.

Die höher siedenden Nebenprodukte enthalten vorzugsweise mindestens ein Cyclohexan-Fragment (Rest), mehr bevorzugt zwei Cyclohexan-Fragmente (Reste), oder mindestens ein Cyclohexan-Fragment (Rest) und mindestens ein aromatisches Fragment (Rest).

Diese umfassen beispielsweise unsubstituierte oder zumindest monoalkylsubstituierte aromatische Amine sowie sekundäre Amine, die als Kondensationsprodukte von zwei oder mehr Molekülen des mindestens einen zumindest monoalkylsubstituierten Diaminocyclohexans (A) unter Abspaltung von Ammoniak entstehen können, wie beispielsweise N¹-(3-Amino-4-methylcyclohexyl)-4-methylcyclohexan-1,3-diamin und dessen Isomere. Des Weiteren umfassen die höher siedenden Nebenprodukte auch Kondensationsprodukte des mindestens einen zumindest monoalkylsubstituierten Diaminocyclohexans (A) mit sonstigen in der Zusammensetzung (ZE) enthaltenen Verbindungen, die Aminogruppen aufweisen, wie beispielsweise mit unsubstituierten oder zumindest monoalkylsubstituierten aromatischen Aminen oder gegebenenfalls unsubstituierten oder zumindest monoalkylsubstituierten Mono-Aminocyclohexanen.

Des Weiteren können bei der Lagerung des mindestens einen zumindest monoalkylsubstituierten Diaminocyclohexans (A) an Luft, im Laufe der Zeit durch Oxidationsreaktionen höher siedende Nebenprodukte entstehen, die an der Verfärbung des mindestens einen zumindest monoalkylsubstituierten Diaminocyclohexans (A) in der Zusammensetzung (ZE) beteiligt sein können. Diese umfassen beispielsweise Iminoverbindungen sowie olefinisch ungesättigte Verbindungen, die durch Oxidation der vorstehend genannten Kondensationsprodukte entstehen können.

Entsprechende Verbindungen umfassen beispielsweise oxidierte Kondensationsprodukte wie N-(3-Imino-4-methylcyclohexyl)-4-methylcyclohex-1-en-1-amin und dessen Isomere sowie oxidierte Kondensationsprodukte des mindestens einen zumindest monoalkylsubstituierten Diaminocyclohexans (A) mit sonstigen in der Zusammensetzung (ZE) enthaltenen Verbindungen, die Aminogruppen aufweisen.

Bevorzugt sind die höher siedenden Nebenprodukte ausgewählt aus unsubstituierten oder zumindest monoalkylsubstituierten aromatischen Aminen oder sekundären Aminen, Iminen und/oder olefinisch ungesättigten Verbindungen, die durch Kondensationsreaktionen sowie gegebenenfalls Oxidationsreaktionen des mindestens einen zumindest monoalkylsubstituierten Diaminocyclohexans (A), gegebenenfalls mit weiteren in der Zusammensetzung (ZE) enthaltenen Aminen, entstehen.

Besonders bevorzugt sind die höher siedenden Nebenprodukte ausgewählt aus N¹-(3-amino-4-methylcyclohexyl)-4-methylcyclohexan-1,3-diamin oder N-(3-imino-4-methylcyclohexyl)-4-methylcyclohex-1-en-1-amin sowie deren Isomere.

In einer Ausführungsform enthält die Zusammensetzung (ZE)

| | |
|---|---|
| 95 bis 99,999 Gew.-% | des mindestens einen zumindest monoalkylsubstituierten Diaminocyclohexans (A), |
| 0,001 bis 5 Gew.-% | höher siedende Verbindungen, |

bezogen auf das Gesamtgewicht der Zusammensetzung (ZE), wobei die Summe aller Komponenten in der Zusammensetzung (ZE) 100 Gew.-% ergibt.

In einer weiteren Ausführungsform enthält die Zusammensetzung (ZE)

| | |
|---|---|
| 94 bis 99,949 Gew.-% | des mindestens einen zumindest monoalkylsubstituierten Diaminocyclohexans (A), |
| 0,05 bis 1 Gew.-% | Wasser, |
| 0,001 bis 5 Gew.-% | höher siedende Verbindungen, |

bezogen auf das Gesamtgewicht der Zusammensetzung (ZE), wobei die Summe aller Komponenten in der Zusammensetzung (ZE) 100 Gew.-% ergibt.

Vorzugsweise werden die in der Zusammensetzung (ZE) enthaltenen weiteren Verbindungen entfernt, bevor die Zugabe des mindestens einen Reduktionsmittels (R) erfolgt (siehe unten).

Im erfindungsgemäßen Verfahren wird der Zusammensetzung (ZE) mindestens ein Reduktionsmittel (R) zugegeben unter Erhalt der Zusammensetzung (ZP).

Die Zugabe des mindestens einen Reduktionsmittels (R) kann dabei nach allen dem Fachmann bekannten Methoden erfolgen und erfolgt vorzugsweise unter Rühren.

Die Zugabe des mindestens einen Reduktionsmittels (R) kann prinzipiell bei beliebigen Temperaturen erfolgen. Vorzugsweise erfolgt die Zugabe des mindestens einen Reduktionsmittels (R) bei niedrigen Temperaturen, bevorzugt im Bereich von 5 bis 60 °C und besonders bevorzugt im Bereich von 10 bis 40 °C.

Die Dauer der Zugabe des mindestens einen Reduktionsmittels (R) kann prinzipiell innerhalb sehr breiter Zeitspannen erfolgen. Die Dauer der Zugabe des mindestens einen Reduktionsmittels (R) befindet sich vorzugsweise im Bereich von 10 Minuten bis 8 Stunden, besonders bevorzugt im Bereich von 15 Minuten bis 5 Stunden und besonders bevorzugt im Bereich von 20 Minuten bis 3 Stunden. Der Fachmann wählt die Dauer der Zugabe des mindestens einen Reduktionsmittels (R) entsprechend aus, um eine homogene Lösung der Zusammensetzung (ZP) zu erhalten.

Die Zusammensetzung (ZP) ist das Gemisch, welches das mindestens eine Reduktionsmittel (R), das mindestens eine zumindest monoalkylsubstituierte Diaminocyclohexan (A) und zusätzlich mindestens 0,05 Gew.-% Wasser aufweist, bezogen auf das Gesamtgewicht der Zusammensetzung (ZP). Folglich beziehen sich sämtliche Angaben bezüglich der Zusammensetzung (ZP) auf das Gemisch nach der Zugabe des mindestens einen Reduktionsmittel (R) sowie gegebenenfalls Wasser.

Das mindestens eine Reduktionsmittel (R) kann genau ein Reduktionsmittel als auch Mischungen von zwei oder mehreren unterschiedlichen Reduktionsmitteln enthalten. Das mindestens eine Reduktionsmittel (R) enthält bevorzugt mindestens eine Komponente, welche Hydridionen enthält.

Bevorzugt enthält das mindestens eine Reduktionsmittel (R) mindestens eine hydridionenhaltige Bor- oder Aluminiumverbindung. Entsprechende Verbindungen sind dem Fachmann prinzipiell bekannt.

Besonders bevorzugt ist das mindestens eine Reduktionsmittel (R) ausgewählt aus Lithiumborhydrid, Natriumborhydrid, Kaliumborhydrid, Natriumcyanoborhydrid, Lithiumaluminiumhydrid, Natriumaluminiumhydrid oder Kaliumaluminiumhydrid. Ganz besonders bevorzugt ist das mindestens eine Reduktionsmittel (R) Natriumborhydrid.

In der Zusammensetzung (ZP) kann das mindestens eine Reduktionsmittel (R) prinzipiell in allen für Stabilisatoren gängigen und dem Fachmann bekannten Mengen enthalten sein. Die Zusammensetzung (ZP) enthält vorzugsweise 0,005 bis 0,2 Gew.-% des mindestens einen Reduktionsmittels (R), bezogen auf das Gesamtgewicht der Zusammensetzung (ZP). Bevorzugt enthält die Zusammensetzung (ZP) 0,007 bis 0,15 Gew.-% und besonders bevorzugt 0,01 bis 0,1 Gew.-% des mindestens einen Reduktionsmittels (R), bezogen auf das Gesamtgewicht der Zusammensetzung (ZP).

Die Zugabe des mindestens einen Reduktionsmittels (R) kann als Feststoff, in einer Lösung (L) oder in einer Suspension (S) erfolgen. Erfolgt die Zugabe des mindestens einen Reduktionsmittels (R) in einer Lösung (L) oder in einer Suspension (S), können die Lösung (L) oder die Suspension (S) prinzipiell jedes beliebige Lösungsmittel enthalten. Vorzugsweise enthalten die Lösung (L) oder die Suspension (S) als Lösungsmittel Wasser, Amine, Ether oder Alkohole, besonders bevorzugt enthalten die Lösung (L) oder die Suspension (S) als Lösungsmittel Wasser.

Das mindestens eine Reduktionsmittel (R) liegt in der Zusammensetzung (ZP) vorzugsweise vollständig gelöst vor. Dies bedeutet, dass die Zusammensetzung (ZP) bevorzugt keine Feststoffpartikel des mindestens einen Reduktionsmittels (R) enthält. Folglich kann das mindestens eine Reduktionsmittel (R) vorzugsweise nicht durch Filtration von der Zusammensetzung (ZP) abgetrennt werden.

Analog dazu liegen Reaktionsprodukte des mindestens einen Reduktionsmittels (R) mit in der Zusammensetzung (ZP) gegebenenfalls enthaltenen, höher siedenden Nebenprodukten oder mit Oxidationsmitteln wie Sauerstoff, vorzugsweise Luftsauerstoff, in der Zusammensetzung (ZP) bevorzugt vollständig gelöst vor. Dies bedeutet, dass die Zusammensetzung (ZP) bevorzugt auch keine Feststoffpartikel der vorstehend genannten Reaktionsprodukte des mindestens einen Reduktionsmittels (R) enthält. Folglich können auch die vorstehend genannten Reaktionsprodukte des mindestens eine Reduktionsmittels (R) vorzugsweise nicht durch Filtration von der Zusammensetzung (ZP) abgetrennt werden.

In einer Ausführungsform wird das mindestens eine Reduktionsmittel (R) in einer Lösung (L) zugegeben, wobei die Lösung (L) vorzugsweise mindestens eine basische Verbindung (B) enthält. In einer weiteren Ausführungsform wird das mindestens eine Reduktionsmittel (R) in einer Suspension (S) zugegeben, wobei die Suspension (S) vorzugsweise mindestens eine basische Verbindung (B) enthält. Sämtliche der nachfolgenden Bevorzugungen für die Lösung (L) gelten für die Suspension (S) entsprechend.

Die Lösung (L) kann genau eine basische Verbindung (B) oder auch Mischungen von zwei oder mehreren verschiedenen basischen Verbindungen (B) enthalten.

Die Menge der mindestens einen basischen Verbindung (B) in der Lösung (L) ist für das erfindungsgemäße Verfahren nicht entscheidend. Vorzugsweise enthält die Lösung (L) 0,1 bis 75 Gew.-% der mindestens einen basischen Verbindung (B), bezogen auf das Gesamtgewicht der Lösung (L). Bevorzugt enthält die Lösung (L) 5 bis 70 Gew.-% und besonders bevorzugt 10 bis 65 Gew.-% der mindestens einen basischen Verbindung (B), bezogen auf das Gesamtgewicht der Lösung (L).

Die mindestens eine basische Verbindung (B) kann prinzipiell jede dem Fachmann bekannte basische Verbindung sein. Bevorzugt ist die mindestens eine basische Verbindung (B) eine basische Alkali- oder Erdalkalimetallverbindung. Besonders bevorzugt ist die mindestens eine basische Verbindung (B) ausgewählt aus Lithiumhydroxid, Natriumhydroxid, Kaliumhydroxid, Magnesiumhydroxid, Calciumhydroxid, Bariumhydroxid, Lithiumcarbonat, Natriumcarbonat, Kaliumcarbonat, Magnesiumcarbonat oder Calciumcarbonat. Ganz besonders bevorzugt ist die mindestens eine basische Verbindung (B) ausgewählt aus Natriumhydroxid, Kaliumhydroxid oder Natriumcarbonat.

In einer bevorzugten Ausführungsform enthält das Gemisch (G) die folgenden Komponenten:
a) 5 bis 20 Gew.-% des mindestens einen Reduktionsmittels (R),
b) 10 bis 65 Gew.-% der mindestens einen basischen Verbindung (B), und
c) 15 bis 85 Gew.-% Wasser,
wobei die Gewichtsanteile der Komponenten a), b) und c) insgesamt 100 Gew.-% ergeben.

Wie vorstehend bereits erwähnt, können vor allem bei der Lagerung des mindestens einen zumindest monoalkylsubstituierten Diaminocyclohexans (A) an Luft im Laufe der Zeit höher siedende Nebenprodukte entstehen, die an der Verfärbung des mindestens einen zumindest monoalkylsubstituierten Diaminocyclohexans (A) beteiligt sind.

Erfindungsgemäß wird deshalb zunächst eine Destillation der Zusammensetzung (ZE) unter Abtrennung höher siedender Nebenprodukte und unter Erhalt einer destillierten Zusammensetzung (DZE) durchgeführt, die das mindestens eine zumindest monoalkylsubstituierte Diaminocyclohexan (A) und gegebenenfalls Wasser enthält, worauf die Zugabe des mindestens einen Reduktionsmittels (R) und gegebenenfalls Wasser zu der destillierten Zusammensetzung (DZE) unter Erhalt der Zusammensetzung (ZP) erfolgt, wobei die Zusammensetzung (ZP) mindestens 0,05 Gew.-% Wasser aufweist, bezogen auf das Gesamtgewicht der Zusammensetzung (ZP).

Der Vollständigkeit halber wird angemerkt, dass in dieser Ausführungsform die Zugabe des mindestens einen Reduktionsmittels (R) zu der destillierten Zusammensetzung (DZE) erfolgt und nicht zu der Zusammensetzung (ZE).

Die Destillation kann nach allen dem Fachmann bekannten und bezogen auf die jeweilige Ausführungsform als technisch angebracht bewerteten Methoden erfolgen.

Die Destillation kann beispielsweise unter anderem an einem Rotationsverdampfer, einer Destillationskolonne, durch Kugelrohrdestillation oder Kurzwegdestillation erfolgen.

Die Destillation kann auch in mehreren Schritten mittels einer oder einer Kombination von verschiedenen Destillationstechniken und kann kontinuierlich oder diskontinuierlich erfolgen.

Die Destillation kann prinzipiell an Luft oder unter Ausschluss von Sauerstoff erfolgen. Um ungewünschte Oxidationsreaktionen durch das mindestens eine zumindest monoalkylsubstituierte Diaminocyclohexan (A) oder weitere in der Zusammensetzung (ZE) enthaltenen Verbindungen während der Destillation zu vermeiden, erfolgt die Destillation vorzugsweise unter Ausschluss von Sauerstoff. "Unter Ausschluss von Sauerstoff" bedeutet im Rahmen der vorliegenden Erfindung, dass der Volumenanteil von Sauerstoff in der Destillationsvorrichtung kleiner als 0,1 Vol.-%, bevorzugt kleiner als 0,1 Vol.-% und besonders bevorzugt kleiner als 0,01 Vol.-% ist, bezogen auf das Gesamtvolumen der Destillationsvorrichtung.

Die Destillation kann prinzipiell bei beliebigen Temperaturen erfolgen. Vorzugsweise erfolgt die Destillation bei einer Temperatur im Bereich von 70 bis 180 °C, bevorzugt im Bereich von 80 bis 170 °C und besonders bevorzugt im Bereich von 90 bis 160 °C.

Die Destillation kann prinzipiell bei beliebigen Drücken erfolgen. Vorzugsweise erfolgt die Destillation bei einem Druck im Bereich von 0,1 bis 500 mbar, bevorzugt im Bereich von 0,5 bis 300 mbar und besonders bevorzugt im Bereich von 1 bis 100 mbar.

In einer bevorzugten Ausführungsform erfolgt die Destillation bei einer Temperatur im Bereich von 70 bis 180 °C, bevorzugt im Bereich von 80 bis 170 °C und besonders bevorzugt im Bereich von 90 bis 160 °C und bei einem Druck im Bereich von 0,1 bis 500 mbar, bevorzugt im Bereich von 0,5 bis 300 mbar und besonders bevorzugt im Bereich von 1 bis 100 mbar.

Die durch das erfindungsgemäße Verfahren erhaltene Zusammensetzung (ZP) enthält mindestens 0,05 Gew.-% Wasser, bezogen auf das Gesamtgewicht der Zusammensetzung (ZP).

Die Zusammensetzung (ZP) enthält 0,05 bis 3 Gew.-%, bevorzugt 0,10 bis 2 Gew.-% und besonders
bevorzugt 0,15 bis 1,5 Gew.-% Wasser, bezogen auf das Gesamtgewicht der Zusammensetzung (ZP).

Prinzipiell kann das Wasser auf beliebige Weise der Zusammensetzung (ZP) zugeführt worden sein.

In einer Ausführungsform ist zumindest ein Teil des in der Zusammensetzung (ZP) enthaltenen Wassers bereits in der Zusammensetzung (ZE) enthalten, wobei die Zusammensetzung (ZE) vorzugsweise einen Wassergehalt von 0,05 bis 1 Gew.-%, bevorzugt von 0,08 bis 0,8 Gew.-% und besonders bevorzugt von 0,1 bis 0,5 Gew.-% aufweist, bezogen auf das Gesamtgewicht der Zusammensetzung (ZE). Wie vorstehend bereits erwähnt, kann das Wasser beispielsweise ein Rückstand aus dem Herstellungsverfahren des mindestens einen zumindest monoalkylsubstituierten Diaminocyclohexans (A) sein.

Erfolgt vor der Zugabe des mindestens einen Reduktionsmittels (R) eine Destillation, so wird das in der Zusammensetzung (ZE) enthaltene Wasser vorzugsweise nicht vollständig abgetrennt. Für den Fall, dass die Zusammensetzung (ZE) einen Wassergehalt von 0,05 bis 1 Gew.-%, bevorzugt von 0,08 bis 0,8 Gew.-% und besonders bevorzugt von 0,1 bis 0,5 Gew.-% aufweist, bezogen auf das Gesamtgewicht der Zusammensetzung (ZE), erfolgt besonders bevorzugt durch die Destillation gar keine Abtrennung von Wasser.

Für den Fall, dass die Zusammensetzung (ZE) einen Wassergehalt von mehr als 1 Gew.-% aufweist, bezogen auf das Gesamtgewicht der Zusammensetzung (ZE), wird durch die Destillation vorzugsweise so viel Wasser abgetrennt, bis die destillierte Zusammensetzung (DZE) höchstens 1 Gew.-%, bevorzugt höchstens 0,8 Gew.-% und besonders bevorzugt 0,5 Gew.-% Wasser enthält, bezogen auf das Gesamtgewicht der Zusammensetzung (DZE).

In einer weiteren Ausführungsform umfasst das erfindungsgemäße Verfahren zusätzlich die Zugabe von Wasser. Die Zugabe von Wasser kann dabei nach allen dem Fachmann bekannten Methoden erfolgen und erfolgt vorzugsweise unter Rühren.

Die Zugabe von Wasser kann gemeinsam mit dem mindestens einen Reduktionsmittel (R) erfolgen oder auch vor oder nach der Zugabe des mindestens einen Reduktionsmittels (R).

Das Wasser, das zugegeben wird, kann prinzipiell beliebiges Wasser sein, beispielsweise demineralisiertes Wasser oder einfach oder mehrfach destilliertes Wasser.

Erfolgt die Zugabe des mindestens einen Reduktionsmittels (R) in einer Lösung (L), die als Lösungsmittel Wasser enthält, oder in einer Suspension (S), die als Lösungsmittel Wasser enthält, kann auf die zusätzliche Zugabe von Wasser prinzipiell verzichtet werden. Bevorzugt erfolgt die Zugabe von Wasser aber auch dann, wenn das mindestens eine Reduktionsmittel (R) in einer Lösung (L) oder in einer Suspension (S) zugegeben wurde.

In einer weiteren Ausführungsform ist zumindest ein Teil des in der Zusammensetzung (ZP) enthaltenen Wassers bereits in der Zusammensetzung (ZE) enthalten und das erfindungsgemäße Verfahren umfasst zusätzlich die Zugabe von Wasser.

Das Gewichtsverhältnis von Wasser zu dem mindestens einen Reduktionsmittel (R) in der Zusammensetzung (ZP) beträgt vorzugsweise mindestens 1:1, bevorzugt mindestens 2:1 und besonders bevorzugt mindestens 4:1.

Des Weiteren beträgt das Gewichtsverhältnis von Wasser zu dem mindestens einen Reduktionsmittel (R) in der Zusammensetzung (ZP) vorzugsweise höchstens 100:1, bevorzugt höchstens 50:1 und besonders bevorzugt höchstens 30:1.

In einer bevorzugten Ausführungsform beträgt das Gewichtsverhältnis von Wasser zu dem mindestens einen Reduktionsmittel (R) in der Zusammensetzung (ZP) vorzugsweise 100:1 bis 1:1, bevorzugt 50:1 bis 2:1 und besonders bevorzugt 30:1 bis 4:1.

Das Wasser liegt in der Zusammensetzung (ZP) vorzugsweise vollständig vermischt vor. Dies bedeutet, dass die Zusammensetzung (ZP) bevorzugt keine separaten Wasserphasen aufweist. Folglich kann das in der Zusammensetzung (ZP) enthaltene Wasser vorzugsweise nicht durch Phasentrennung von der Zusammensetzung (ZP) abgetrennt werden.

Ein weiterer Gegenstand der vorliegenden Erfindung ist die Zusammensetzung (ZP), die nach dem erfindungsgemäßen Verfahren hergestellt wird.

Die Zusammensetzung (ZP) enthält vorzugsweise die folgenden Komponenten:

| | |
|---|---|
| 96,8 bis 99,945 Gew.-% | mindestens eines zumindest monoalkylsubstituierten Diaminocyclohexans, |
| 0,005 bis 0,2 Gew.-% | mindestens eines Reduktionsmittels (R) und |
| 0,05 bis 3 Gew.-% | Wasser. |

Die durch das erfindungsgemäße Verfahren stabilisierten zumindest monoalkylsubstituierten Diaminocyclohexane können als Synthesebaustein für die Herstellung von Tensiden, Arznei- und Pflanzenschutzmitteln, Stabilisatoren, Lichtschutzmitteln, Polymeren, Isocyanaten, Härtern für Epoxyharze, Katalysatoren für Polyurethane, Zwischenprodukte zur Herstellung quarternärer Ammonium-verbindungen, Weichmachern, Korrosionsinhibitoren, Kunstharzen, Ionenaustauschern, Textilhilfsmitteln, Farbstoffen, Vulkanisationsbeschleunigern, Emulgatoren und/oder als Ausgangssubstanzen für die Herstellung von Harnstoffen und Polyharnstoffen verwendet werden.

Ein weiterer Gegenstand der vorliegenden Erfindung ist somit auch die Verwendung der Zusammensetzung (ZP) für die Herstellung von Tensiden, Arznei- und Pflanzenschutzmitteln, Stabilisatoren, Lichtschutzmitteln, Polymeren, Isocyanaten, Härtern für Epoxyharze, Katalysatoren für Polyurethane, Zwischenprodukte zur Herstellung quarternärer Ammoniumverbindungen, Weichmachern, Korrosionsinhibitoren, Kunstharzen, Ionenaustauschern, Textilhilfsmitteln, Farbstoffen, Vulkanisationsbeschleunigern, Emulgatoren und/oder als Ausgangssubstanzen für die Herstellung von Harnstoffen und Polyharnstoffen.

Insbesondere können 1,3-Diamino-2-methylcyclohexan und 1,3-Diamino-4-methylcyclohexan als Monomerbausteine für Polyamide, als Härter für Epoxyharze oder als Ausgangsprodukte für die Herstellung der entsprechenden Isocyanate verwendet werden.

Die folgenden Beispiele sollen die vorliegende Erfindung weiter verdeutlichen, ohne aber die vorliegende Erfindung darauf zu beschränken.

Die Ermittlung der Hazen-Farbzahl gemäß APHA in den nachfolgenden Beispielen erfolgt gemäß DIN EN ISO 6271 und wird in einem Farbmessgerät der Firma Lange (LICO 400) durchgeführt. Die Bestimmung der Hazen-Farbzahl erfolgt in Lange LZM 130 50mm Einmalküvetten aus Kunststoff. Die vermessenen Proben haben jeweils eine Masse von 60 g.

### Beispiel 1

Proben einer undestillierten Zusammensetzung (ZE), die ein Gemisch aus 1,3-Diamino-2-methylcyclohexan und 1,3-Diamino-4-methylcyclohexan sowie 0,15 Gew.-% Wasser enthält, werden in 250 mL Schraubdeckelgläschen gefüllt, mit Natriumborhydrid und gegebenenfalls Wasser versetzt unter Erhalt einer Zusammensetzung (ZP) und in einem Wärmeschrank bei 80°C gelagert (siehe Versuche 1 und 2 in Tabelle 1).

Ebenso werden 895 g der Zusammensetzung (ZE) bei 1 mbar und 104 °C (Kopftemperatur) über eine Destillationsbrücke destilliert. Es werden 805 g einer destillierten Zusammensetzung (DZE) erhalten, welche ungefähr 0,15 Gew.-% Wasser enthält. Proben dieser destillierten Zusammensetzung (DZE) werden ebenfalls in 250 mL Schraubdeckelgläschen gefüllt, mit Natriumborhydrid und gegebenenfalls Wasser versetzt unter Erhalt einer Zusammensetzung (ZP) und in einem Wärmeschrank bei 80°C gelagert (siehe Versuche 3 bis 9 in Tabelle 1).

Als Referenz werden eine undestillierte und nicht mit Natriumborhydrid versetzte Probe (V1) sowie eine destillierte und nicht mit Natriumborhydrid versetzte Probe (V2) gelagert und vermessen. Alle Proben werden regelmäßig geöffnet, belüftet und pro Messung ca. 6g für die Farbzahlbestimmung entnommen.

Die entsprechenden experimentellen Daten sind in Tabelle 1 wiedergegeben. In den Spalten ist die Hazen-Farbzahl zu den jeweils in der obersten Zeile genannten Zeiten eingetragen.

**Tabelle 1**

| Versuch | NaBH₄ [Gew.-%] | Wasserzugabe [Gew.-%] | 0h | 24 h | 48h | 72h | 96h | 168 h | 192 h | 240h |
|---|---|---|---|---|---|---|---|---|---|---|
| V1 | - | - | 12 | 29 | 48 | 78 | 149 | 318 | 683 | >1000 |
| V2 | - | - | 0 | 0 | 5 | 15 | 31 | 89 | 204 | 509 |
| 1 | 0,05 | - | 12 | 48 | 77 | 103 | 106 | 220 | 282 | 385 |
| 2 | 0,05 | + 0,3 | 12 | 29 | 54 | 79 | 49 | 66 | 135 | 261 |
| 3 | 0,1 | + 0,5 | 0 | 58 | 92 | 122 | 128 | 96 | 72 | 67 |
| 4 | 0,1 | + 1,0 | 0 | 15 | 38 | 45 | 54 | 22 | 23 | 74 |
| 5 | 0,1 | + 1,5 | 0 | 10 | 17 | 12 | 44 | 25 | 68 | 178 |
| 6 | 0,05 | + 0,2 | 0 | 51 | 92 | 100 | 92 | 127 | 140 | 188 |
| 7 | 0,05 | + 0,5 | 0 | 56 | 69 | 75 | 85 | 56 | 59 | 167 |
| 8 | 0,05 | + 1,0 | 0 | 12 | 28 | 58 | 49 | 23 | 34 | 226 |
| 9 | 0,01 | - | 0 | 15 | 24 | 32 | 32 | 22 | 29 | 121 |

Die in Tabelle 1 angeführten experimentellen Daten zeigen, dass die Hazen-Farbzahl der Zusammensetzung (ZP) langfristig deutlich niedriger gehalten werden kann, wenn das mindestens eine Reduktionsmittel (R) sowie gegebenenfalls Wasser eingesetzt werden (Versuche 1 bis 9). Wurde die Zusammensetzung (ZE) zunächst destilliert und anschließend das mindestens eine Reduktionsmittel (R) sowie gegebenenfalls Wasser zugegeben (siehe Versuche 3 bis 9), ist die Hazen-Farbzahl über einen längeren Zeitraum geringer als bei undestillierten stabilisierten Zusammensetzungen (ZP) (siehe Versuche 1 und 2).

### Beispiel 2

Proben einer undestillierten Zusammensetzung (ZE), die ein Gemisch aus 1,3-Diamino-2-methylcyclohexan und 1,3-Diamino-4-methylcyclohexan sowie 0,15 Gew.-% Wasser enthält, werden in 250 mL Schraubdeckelgläschen gefüllt, mit Natriumborhydrid bzw. Borol^{™} (12,5 Gew.-% Natriumborhydrid in einer wässrigen, 14 molaren Lösung von Natriumhydroxid, zu beziehen von Dow Chemicals) und gegebenenfalls Wasser versetzt unter Erhalt einer Zusammensetzung (ZP) und in einem Wärmeschrank bei 80°C gelagert (siehe Versuche 1 und 2 in Tabelle 2).

Ebenso wurden 860 g der Zusammensetzung (ZE) bei 1 mbar und 104 °C (Kopftemperatur) über eine Destillationsbrücke destilliert. Es werden 730 g einer destillierten Zusammensetzung (DZE) erhalten, welche ungefähr 0,15 Gew.-% Wasser enthält. Proben dieser destillierten Zusammensetzung (DZE) werden ebenfalls in 250 mL Schraubdeckelgläschen gefüllt, mit Natriumborhydrid bzw. Borol und gegebenenfalls Wasser versetzt unter Erhalt einer Zusammensetzung (ZP) und in einem Wärmeschrank bei 80°C gelagert (siehe Versuche 3 bis 7 in Tabelle 2).

Als Referenz wird eine destillierte und nicht mit einem Reduktionsmittel (R) versetzte Probe (Versuch V3) gelagert und vermessen. Da die undestillierte Zusammensetzung (ZE) identisch zu der undestillierten Zusammensetzung (ZE) aus Beispiel 1 ist, entspricht die Referenzmessung der undestillierten und nicht mit einem Reduktionsmittel (R) versetzten Probe dem Versuch V1 aus Beispiel 1. Alle Proben werden regelmäßig geöffnet, belüftet und pro Messung ca. 6g für die Farbzahlbestimmung entnommen.

In den Versuchen 1 bis 5 wird Borol^{™} als Reduktionsmittel (R) eingesetzt und in den Versuchen 6 und 7 wird Natriumborhydrid als Reduktionsmittel (R) eingesetzt. Die Angaben zum Gewichtsanteil des mindestens einen Reduktionsmittels (R) in der nachfolgenden Tabelle 2 beziehen sich stets auf die eingesetzte Menge an Natriumborhydrid. Wird beispielsweise Borol^{™} als Reduktionsmittel (R) verwendet, so bedeutet die Bezeichnung "0,02 Gew.-%", dass eine bestimmte Menge Borol^{™} eingesetzt wird bis 0,02 Gew.-% Natriumborhydrid mittels Borol^{™} zugegeben wurden.

Die entsprechenden experimentellen Daten sind in Tabelle 2 wiedergegeben. In den Spalten ist die Hazen-Farbzahl zu den jeweils in der obersten Zeile genannten Zeiten eingetragen.

**Tabelle 2**

| Versuch | Reduktionsmittel (R) [Gew.-%] | Wasserzugabe [Gew.-%] | 0h | 24 h | 48h | 72h | 96h | 168 h | 192 h | 240h |
|---|---|---|---|---|---|---|---|---|---|---|
| V1 | - | - | 12 | 29 | 48 | 78 | 149 | 318 | 683 | >1000 |
| V3 | - | - | 0 | 1 | 3 | 12 | 30 | 102 | 251 | 538 |
| 1 | 0,02 | - | 0 | 71 | 116 | 155 | 208 | 368 | 487 | 653 |
| 2 | 0,04 | - | 0 | 105 | 97 | 97 | 130 | 412 | 360 | 501 |
| 3 | 0,01 | - | 0 | 8 | 14 | 15 | 12 | 24 | 33 | 152 |
| 4 | 0,02 | - | 0 | 10 | 17 | 22 | 16 | 20 | 35 | 218 |
| 5 | 0,04 | - | 0 | 32 | 63 | 72 | 83 | 67 | 83 | 185 |
| 6 | 0,02 | + 0,1 | 0 | 19 | 38 | 39 | 52 | 34 | 41 | 115 |
| 7 | 0,04 | +0,2 | 0 | 39 | 89 | 94 | 100 | 75 | 70 | 177 |

Die in Tabelle 2 angeführten experimentellen Daten zeigen, dass die Hazen-Farbzahl der Zusammensetzung (ZP) langfristig deutlich niedriger gehalten werden kann, wenn das mindestens eine Reduktionsmittel (R) sowie gegebenenfalls Wasser eingesetzt werden (Versuche 1 bis 7). Wird die Zusammensetzung (ZE) zunächst destilliert und anschließend das mindestens eine Reduktionsmittel (R) sowie gegebenenfalls Wasser zugegeben (siehe Versuche 3 bis 7), ist die Hazen-Farbzahl über einen längeren Zeitraum geringer als bei undestillierten stabilisierten Zusammensetzungen (ZP) (siehe Versuche 1 und 2).

### Beispiel 3

449 g einer undestillierten Zusammensetzung (ZE), die 99,76 Gew.-% eines Gemisches aus 1,3-Diamino-2-methylcyclohexan und 1,3-Diamino-4-methylcyclohexan und 0,24 Gew.-% weiterer Verbindungen enthält, werden bei 70 mbar und 136 °C (Kopftemperatur) destilliert. Es werden 51 g einer ersten Fraktion erhalten und abgetrennt und anschließend werden 343 g einer destillierten Zusammensetzung (DZE) erhalten. Die destillierte Zusammensetzung (DZE) enthält dabei kein Wasser.

Proben dieser destillierten Zusammensetzung (DZE) werden in 250 mL Schraubdeckelgläschen gefüllt, mit einem Reduktionsmittel (R) versetzt und in einem Wärmeschrank bei 80°C gelagert.

Als Referenz wird eine destillierte und nicht mit einem Reduktionsmittel (R) versetzte Probe (Versuch V1) gelagert und vermessen. Alle Proben werden regelmäßig geöffnet, belüftet und pro Messung ca. 6 g für die Farbzahlbestimmung entnommen.

In Versuch V2 wird Borol^{™} (12,5 Gew.-% Natriumborhydrid in einer wässrigen, 14 molaren Lösung von Natriumhydroxid, zu beziehen von Dow Chemicals) als Reduktionsmittel (R) eingesetzt und in Versuch V3 wird Natriumborhydrid als Reduktionsmittel (R) eingesetzt. Die Angaben zu den Gewichtsanteilen des mindestens einen Reduktionsmittels (R) in der nachfolgenden Tabelle 3 beziehen sich stets auf die eingesetzte Menge an Natriumborhydrid. Wird Borol^{™} als Reduktionsmittel (R) verwendet, so bedeutet die Bezeichnung "0,1 Gew.-%" beispielsweise, dass eine bestimmte Menge Borol^{™} eingesetzt wird bis 0,1 Gew.-% Natriumborhydrid mittels Borol^{™} zugegeben wurden. Bei der Verwendung von Borol^{™} liegt der Wassergehalt der Proben stets unter 0,1 Gew.-%.

Die entsprechenden experimentellen Daten sind in Tabelle 3 wiedergegeben. In den Spalten ist die Hazen-Farbzahl zu den jeweils in der obersten Zeile genannten Zeiten eingetragen.

**Tabelle 3**

| Versuch | Reduktions-mittel (R) [Gew.-%] | 0h | 24 h | 48h | 72h | 96h | 168 h | 192 h | 240h |
|---|---|---|---|---|---|---|---|---|---|
| V1 | - | 0 | 0 | 3 | 11 | 32 | 78 | 159 | 305 |
| V2 | 0,1 | 0 | 3 | 9 | 45 | 104 | 253 | 402 | 791 |
| V3 | 0,1 | 1 | 96 | 123 | 195 | 260 | 339 | 837 | >1000 |

Die in Tabelle 3 angeführten experimentellen Daten zeigen, dass für eine langfristig geringe Hazen-Farbzahl neben dem mindestens einen Reduktionsmittel (R) mindestens 0,05 Gew.-% Wasser, bezogen auf das Gesamtgewicht der Zusammensetzung (ZP), in der Zusammensetzung (ZP) vorliegen müssen.

### Beispiel 4

Proben einer undestillierten Zusammensetzung (ZE), die ein Gemisch aus 1,3-Diamino-2-methylcyclohexan und 1,3-Diamino-4-methylcyclohexan sowie 0,15 Gew.-% Wasser enthält, werden in 250 mL Schraubdeckelgläschen gefüllt, mit Natriumborhydrid oder einer Natriumborhydrid-Stammlösung versetzt unter Erhalt einer Zusammensetzung (ZP) und in einem Wärmeschrank bei 60°C gelagert (siehe Versuche 1 und 2 in Tabelle 4).

Als Referenz wird eine undestillierte und nicht mit Natriumborhydrid versetzte Probe (V1) gelagert und vermessen. Alle Proben werden regelmäßig geöffnet, belüftet und pro Messung ca. 6 g für die Farbzahlbestimmung entnommen.

Die Natriumborhydrid-Stammlösung wird folgendermaßen hergestellt: In einem 100 mL Rührkolben mit Blasenzähler werden unter Stickstoff 2 g NaBH₄-Pulver vorgelegt und anschließend 18 g der Zusammensetzung (ZE) dazu addiert. Nach einer Stunde Rühren ist das NaBH₄ bis auf ein paar Kristalle fast vollständig gelöst. Die überstehende Lösung wird als NaBH₄-Stammlösung verwendet.

Die entsprechenden experimentellen Daten sind in Tabelle 4 wiedergegeben. In den Spalten ist die Hazen-Farbzahl zu den jeweils in der obersten Zeile genannten Zeiten eingetragen.

**Tabelle 4**

| Versuch | NaBH₄ [Gew.-%] | Zugabe Stammlösung [Gew.-%] | 0h | 24 h | 48h | 72h | 144h | 168 h | 192 h | 312 h | 336 h | 360 h | 384 h | 408 h |
|---|---|---|---|---|---|---|---|---|---|---|---|---|---|---|
| V1 | - | - | 21 | 27 | 36 | 47 | 89 | 106 | 128 | 233 | 277 | 326 | 386 | 454 |
| 1 | 0,01 | - | 21 | 41 | 36 | 35 | 48 | 57 | 67 | 107 | 122 | 141 | 171 | 183 |
| 2 | - | 0,01 | 21 | 38 | 32 | 32 | 51 | 60 | 70 | 111 | 129 | 147 | 172 | 198 |

Die in Tabelle 4 angeführten experimentellen Daten zeigen, dass die Hazen-Farbzahl der Zusammensetzung (ZP) langfristig deutlich niedriger gehalten werden kann, wenn Natriumborhydrid oder einer Natriumborhydrid-Stammlösung eingesetzt werden (Versuche 1 und 2).

## Patentansprüche

1. Verfahren zur Stabilisierung von zumindest monoalkylsubstituierten Diaminocyclohexanen, umfassend die Zugabe mindestens eines Reduktionsmittels (R) sowie gegebenenfalls Wasser zu einer Zusammensetzung (ZE), welche mindestens ein zumindest monoalkylsubstituiertes Diaminocyclohexan (A) sowie gegebenenfalls Wasser enthält, unter Erhalt einer Zusammensetzung (ZP), wobei die Zusammensetzung (ZP) das mindestens eine Reduktionsmittel (R), das mindestens eine zumindest monoalkylsubstituierte Diaminocyclohexan (A) und zusätzlich mindestens 0,05 Gew.-% Wasser aufweist, bezogen auf das Gesamtgewicht der Zusammensetzung (ZP), **dadurch gekennzeichnet, dass** das mindestens eine zumindest monoalkylsubstituierte Diaminocyclohexan (A) aus einer der Verbindungen gemäß den allgemeinen Formeln (I), (II) oder (III) ausgewählt ist 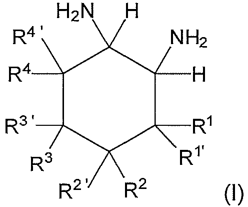 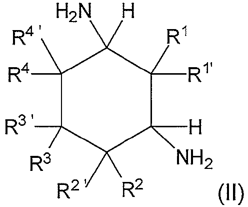 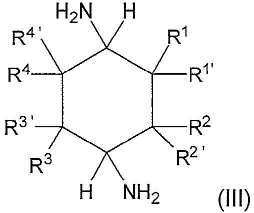 in denen
R₁, R₁', R₂, R₂', R₃, R₃', R₄ und R₄' unabhängig voneinander ausgewählt sind aus H und C₁-C₄-Alkyl, wobei mindestens ein Rest R₁, R₁', R₂, R₂', R₃, R₃', R₄ oder R₄' C₁-C₄-Alkyl ist,
**dadurch gekennzeichnet, dass** zunächst eine Destillation der Zusammensetzung (ZE) unter Abtrennung höher siedender Nebenprodukte und unter Erhalt einer destillierten Zusammensetzung (DZE), die das mindestens eine zumindest monoalkylsubstituierte Diaminocyclohexan (A) und gegebenenfalls Wasser enthält, durchgeführt wird, worauf die Zugabe des mindestens einen Reduktionsmittels (R) und gegebenenfalls Wasser zu der destillierten Zusammensetzung (DZE) unter Erhalt der Zusammensetzung (ZP) erfolgt, wobei die Zusammensetzung (ZP) 0,05 bis 3 Gew.-% Wasser enthält, bezogen auf das Gesamtgewicht der Zusammensetzung (ZP).

2. Verfahren gemäß Anspruch 1, **dadurch gekennzeichnet, dass** das mindestens eine zumindest monoalkylsubstituierte Diaminocyclohexan (A) ausgewählt ist aus 1,3-Diamino-4-methylcyclohexan oder 1,3-Diamino-2-methylcyclohexan.

3. Verfahren gemäß einem der Ansprüche 1 bis 2, **dadurch gekennzeichnet, dass** die Zusammensetzung (ZE) mindestens 85 Gew.-%, bevorzugt mindestens 90 Gew.-% und besonders bevorzugt mindestens 95 Gew.-% des mindestens einen zumindest monoalkylsubstituierten Diaminocyclohexans (A) enthält, bezogen auf das Gesamtgewicht der Zusammensetzung (ZE).

4. Verfahren gemäß einem der Ansprüche 1 bis 3, **dadurch gekennzeichnet, dass** das mindestens eine Reduktionsmittel (R) ausgewählt ist aus Lithiumborhydrid, Natriumborhydrid, Kaliumborhydrid, Natriumcyanoborhydrid, Lithiumaluminiumhydrid, Natriumaluminiumhydrid oder Kaliumaluminiumhydrid;
bevorzugt ist das mindestens eine Reduktionsmittel (R) Natriumborhydrid.

5. Verfahren gemäß einem der Ansprüche 1 bis 4, **dadurch gekennzeichnet, dass** die Zusammensetzung (ZP) 0,005 bis 0,2 Gew.-%, bevorzugt 0,007 bis 0,15 Gew.-% und besonders bevorzugt 0,01 bis 0,1 Gew.-% des mindestens einen Reduktionsmittels (R) enthält, bezogen auf das Gesamtgewicht der Zusammensetzung (ZP).

6. Verfahren gemäß einem der Ansprüche 1 bis 5, **dadurch gekennzeichnet, dass** die Zugabe des mindestens einen Reduktionsmittels (R) als Feststoff, in einer Lösung (L) oder in einer Suspension (S) erfolgt.

7. Verfahren gemäß Anspruch 6, **dadurch gekennzeichnet, dass** das Gemisch (G) mindestens eine basische Verbindung (B) enthält, die ausgewählt ist aus Lithiumhydroxid, Natriumhydroxid, Kaliumhydroxid, Ammoniumhydroxid, Magnesiumhydroxid, Calciumhydroxid, Bariumhydroxid, Lithiumcarbonat, Natriumcarbonat, Kaliumcarbonat, Magnesiumcarbonat oder Calciumcarbonat.

8. Verfahren gemäß Anspruch 6 oder 7, **dadurch gekennzeichnet, dass** die Lösung (L) die folgenden Komponenten enthält:
a) 5 bis 20 Gew.-% des mindestens einen Reduktionsmittels (R),
b) 10 bis 65 Gew.-% der mindestens einen basischen Verbindung (B), und
c) 15 bis 85 Gew.-% Wasser,
wobei die Gewichtsanteile der Komponenten a), b) und c) insgesamt 100 Gew.-% ergeben.

9. Verfahren gemäß der Ansprüche 1 bis 8, **dadurch gekennzeichnet, dass**
i) die Destillation bei einer Temperatur im Bereich von 70 bis 180 °C, bevorzugt im Bereich von 80 bis 170 °C und besonders bevorzugt im Bereich von 90 bis 160 °C erfolgt, und/oder
ii) die Destillation bei einem Druck im Bereich von 0,1 bis 500 mbar, bevorzugt im Bereich von 0,5 bis 300 mbar und besonders bevorzugt im Bereich von 1 bis 100 mbar erfolgt.

10. Verfahren gemäß einem der Ansprüche 1 bis 9, **dadurch gekennzeichnet, dass**
i) zumindest ein Teil des in der Zusammensetzung (ZP) enthaltenen Wassers bereits in der Zusammensetzung (ZE) enthalten ist, wobei die Zusammensetzung (ZE) einen Wassergehalt von 0,05 bis 1 Gew.-%, bevorzugt von 0,08 bis 0,8 Gew.-% und besonders bevorzugt von 0,1 bis 0,5 Gew.-% aufweist, bezogen auf das Gesamtgewicht der Zusammensetzung (ZE), und/oder
ii) das Verfahren zusätzlich die Zugabe von Wasser umfasst, und/oder
iii) zumindest ein Teil des in der Zusammensetzung (ZP) enthaltenen Wassers gemeinsam mit dem mindestens einen Reduktionsmittel (R) zugegeben wird.

11. Verfahren gemäß einem der Ansprüche 1 bis 10, **dadurch gekennzeichnet, dass** die Zusammensetzung (ZP) 0,10 bis 2 Gew.-% und bevorzugt 0,15 bis 1,5 Gew.-% Wasser enthält, bezogen auf das Gesamtgewicht der Zusammensetzung (ZP).

12. Verfahren gemäß einem der Ansprüche 1 bis 11, **dadurch gekennzeichnet, dass** das Gewichtsverhältnis von Wasser zu dem mindestens einen Reduktionsmittel (R) in der Zusammensetzung (ZP) 100:1 bis 1:1, bevorzugt 50:1 bis 2:1 und besonders bevorzugt 30:1 bis 4:1 beträgt.

13. Zusammensetzung (ZP), hergestellt nach dem Verfahren gemäß einem der Ansprüche 1 bis 12.

14. Verwendung der Zusammensetzung (ZP) gemäß Anspruch 13 für die Herstellung von Tensiden, Arznei- und Pflanzenschutzmitteln, Stabilisatoren, Lichtschutzmitteln, Polymeren, Isocyanaten, Härtern für Epoxyharze, Katalysatoren für Polyurethane, Zwischenprodukte zur Herstellung quarternärer Ammonium-verbindungen, Weichmachern, Korrosionsinhibitoren, Kunstharzen, lonenaus-tauschern, Textilhilfsmitteln, Farbstoffen, Vulkanisationsbeschleunigern, Emulgatoren und/oder als Ausgangssubstanzen für die Herstellung von Harnstoffen und Polyharnstoffen.

## Claims

1. A process for stabilizing at least monoalkyl-substituted diaminocyclohexanes comprising addition of at least one reductant (R) and optionally water to a composition (ZE) comprising at least one at least monoalkyl-substituted diaminocyclohexane (A) and optionally water to obtain a composition (ZP), wherein the composition (ZP) comprises the at least one reductant (R), the at least one at least monoalkyl-substituted diaminocyclohexane (A) and additionally at least 0.05% by weight of water based on the total weight of the composition (ZP), wherein the at least one at least monoalkyl-substituted diaminocyclohexane (A) is selected from one of the compounds according to general formulae (I), (II) or (III) 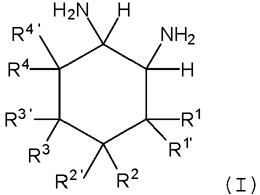 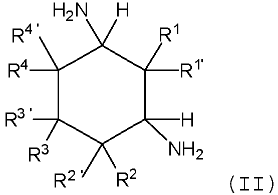 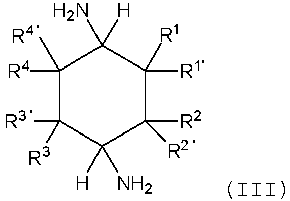 in which
R₁, R₁', R₂, R₂', R₃, R₃', R₄ and R₄' are independently of one another selected from H and C₁-C₄-alkyl, wherein at least one radical R₁, R₁', R₂, R₂', R₃, R₃', R₄ or R₄' is C₁-C₄-alkyl.
wherein said process comprises initially performing a distillation of the composition (ZE) to remove higher boiling byproducts and to obtain a distilled composition (DZE) comprising the at least one at least monoalkyl-substituted diaminocyclohexane (A) and optionally water followed by the addition of the at least one reductant (R) and optionally water to the distilled composition (DZE) to obtain the composition (ZP), wherein the composition (ZP) comprises 0.05 to 3% by weight of water based on the total weight of the composition (ZP).

2. The process according to claim 1, wherein the at least one at least monoalkyl-substituted diaminocyclohexane (A) is selected from 1,3-diamino-4-methylcyclohexane or 1,3-diamino-2-methylcyclohexane.

3. The process according to any of claims 1 to 2, wherein the composition (ZE) comprises at least 85% by weight, preferably at least 90% by weight and particularly preferably at least 95% by weight of the at least one at least monoalkyl-substituted diaminocyclohexane (A) based on the total weight of the composition (ZE).

4. The process according to any of claims 1 to 3, wherein the at least one reductant (R) is selected from lithium borohydride, sodium borohydride, potassium borohydride, sodium cyanoborohydride, lithium aluminum hydride, sodium aluminum hydride or potassium aluminum hydride;
the at least one reductant (R) is preferably sodium borohydride.

5. The process according to any of claims 1 to 4, wherein the composition (ZP) comprises 0.005 to 0.2% by weight, preferably 0.007 to 0.15% by weight and particularly preferably 0.01 to 0.1% by weight of the at least one reductant (R) based on the total weight of the composition (ZP).

6. The process according to any of claims 1 to 5, wherein the at least one reductant (R) is added in the form of a solid, in a solution (L) or in a suspension (S) .

7. The process according to claim 6, wherein the mixture (G) comprises at least one basic compound (B) selected from lithium hydroxide, sodium hydroxide, potassium hydroxide, ammonium hydroxide, magnesium hydroxide, calcium hydroxide, barium hydroxide, lithium carbonate, sodium carbonate, potassium carbonate, magnesium carbonate or calcium carbonate.

8. The process according to claim 6 or 7, wherein the solution (L) comprises the following components:
a) 5% to 20% by weight of the at least one reductant (R) ,
b) 10% to 65% by weight of the at least one basic compound (B) and
c) 15% to 85% by weight of water,
wherein the weight fractions of the components a), b) and c) altogether sum to 100% by weight.

9. The process according to claims 1 to 8, wherein
i) the distillation is effected at a temperature in the range from 70°C to 180°C, preferably in the range from 80°C to 170°C and particularly preferably in the range from 90°C to 160°C, and/or
ii) the distillation is effected at a pressure in the range from 0.1 to 500 mbar, preferably in the range from 0.5 to 300 mbar and particularly preferably in the range from 1 to 100 mbar.

10. The process according to any of claims 1 to 9, wherein
i) at least a portion of the water present in the composition (ZP) is already present in the composition (ZE), wherein the composition (ZE) comprises a water content of 0.05% to 1% by weight, preferably of 0.08% to 0.8% by weight and particularly preferably of 0.1% to 0.5% by weight based on the total weight of the composition (ZE), and/or
ii) the process additionally comprises the addition of water, and/or
iii) at least a portion of the water present in the composition (ZP) is added together with the at least one reductant (R).

11. The process according to any of claims 1 to 10, wherein the composition (ZP) comprises 0.10% to 2% by weight and preferably 0.15% to 1.5% by weight of water based on the total weight of the composition (ZP).

12. The process according to any of claims 1 to 11, wherein the weight ratio of water to the at least one reductant (R) in the composition (ZP) is 100:1 to 1:1, preferably 50:1 to 2:1 and particularly preferably 30:1 to 4:1.

13. A composition (ZP) produced by the process according to any of claims 1 to 12.

14. The use of the composition (ZP) according to claim 13 for the production of surfactants, pharmaceutical and plant protection products, stabilizers, light stabilizers, polymers, isocyanates, hardeners for epoxy resins, catalysts for polyurethanes, intermediates for producing quaternary ammonium compounds, plasticizers, corrosion inhibitors, synthetic resins, ion exchangers, textile auxiliaries, dyes, vulcanization accelerators, emulsifiers and/or as starting materials for the production of ureas and polyureas.

## Revendications

1. Procédé pour la stabilisation de diaminocyclohexanes au moins monosubstitués par alkyle, comprenant l'ajout d'au moins un agent réducteur (R) ainsi que le cas échéant d'eau à une composition (ZE), qui contient au moins un diaminocyclohexane au moins monosubstitué par alkyle (A) ainsi que le cas échéant de l'eau, avec obtention d'une composition (ZP), la composition (ZP) présentant ledit au moins un agent réducteur (R), ledit au moins un diaminocyclohexane au moins monosubstitué par alkyle (A) et en outre au moins 0,05% en poids d'eau, par rapport au poids total de la composition (ZP), **caractérisé en ce que** ledit au moins un diaminocyclohexane au moins monosubstitué par alkyle (A) est choisi parmi l'un des composés selon les formules générales (I), (II) ou (III) 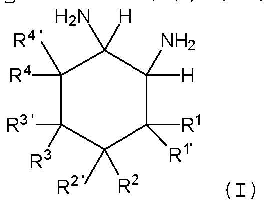 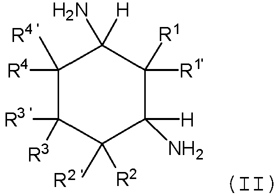 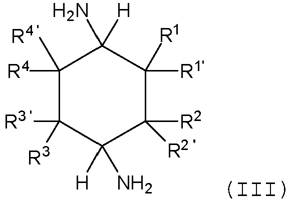 dans lesquelles
R₁, R₁', R₂, R₂', R₃, R₃', R₄ et R₄' sont choisis, indépendamment les uns des autres, parmi H et C₁-C₄-alkyle, au moins un radical R₁, R₁', R₂, R₂', R₃, R₃', R₄ ou R₄' représentant C₁-C₄-alkyle,
**caractérisé en ce que** l'on effectue d'abord une distillation de la composition (ZE) avec séparation de sous-produits à point d'ébullition plus élevé et avec obtention d'une composition distillée (DZE) qui contient ledit au moins un diaminocyclohexane au moins monosubstitué par alkyle (A) et le cas échéant de l'eau, suite à quoi l'ajout dudit au moins un agent réducteur (R) et le cas échéant d'eau à la composition distillée (DZE) est effectué avec obtention de la composition (ZP), la composition (ZP) contenant 0,05 % à 3% en poids d'eau, par rapport au poids total de la composition (ZP).

2. Procédé selon la revendication 1, **caractérisé en ce que** ledit au moins un diaminocyclohexane au moins monosubstitué par alkyle (A) est choisi parmi le 1,3-diamino-4-méthylcyclohexane ou le 1,3-diamino-2-méthylcyclohexane.

3. Procédé selon l'une des revendications 1 ou 2, **caractérisé en ce que** la composition (ZE) contient au moins 85% en poids, de préférence au moins 90% en poids et de manière particulièrement préférée au moins 95% en poids dudit au moins un diaminocyclohexane au moins monosubstitué par alkyle (A), par rapport au poids total de la composition (ZE).

4. Procédé selon l'une des revendications 1 à 3, **caractérisé en ce que** ledit au moins un agent réducteur (R) est choisi parmi le borohydrure de lithium, le borohydrure de sodium, le borohydrure de potassium, le cyanoborohydrure de sodium, l'hydrure de lithium-aluminium, l'hydrure de sodium-aluminium ou l'hydrure de potassium-aluminium ;
de préférence, ledit au moins un agent réducteur (R) est le borohydrure de sodium.

5. Procédé selon l'une des revendications 1 à 4, **caractérisé en ce que** la composition (ZP) contient 0,005 à 0,2% en poids, de préférence 0,007 à 0,15% en poids et de manière particulièrement préférée 0,01 à 0,1% en poids dudit au moins un agent réducteur (R), par rapport au poids total de la composition (ZP).

6. Procédé selon l'une des revendications 1 à 5, **caractérisé en ce que** l'ajout dudit au moins un agent réducteur (R) est effectué sous forme solide, dans une solution (L) ou dans une suspension (S).

7. Procédé selon la revendication 6, **caractérisé en ce que** le mélange (G) contient au moins un composé basique (B) qui est choisi parmi l'hydroxyde de lithium, l'hydroxyde de sodium, l'hydroxyde de potassium, l'hydroxyde d'ammonium, l'hydroxyde de magnésium, l'hydroxyde de calcium, l'hydroxyde de baryum, le carbonate de lithium, le carbonate de sodium, le carbonate de potassium, le carbonate de magnésium ou le carbonate de calcium.

8. Procédé selon la revendication 6 ou 7, **caractérisé en ce que** la solution (L) contient les composants suivants :
a) 5 à 20% en poids d'au moins un agent réducteur (R),
b) 10 à 65% en poids dudit au moins un composé basique (B) et
c) 15 à 85% en poids d'eau,
les proportions en poids des composants a), b) et c) valant au total 100% en poids.

9. Procédé selon les revendications 1 à 8, **caractérisé en ce que**
i) la distillation est effectuée à une température dans la plage de 70 à 180°C, de préférence dans la plage de 80 à 170°C et de manière particulièrement préférée dans la plage de 90 et 160°C et/ou
ii) la distillation est effectuée à une pression dans la plage de 0,1 à 500 mbars, de préférence dans la plage de 0,5 à 300 mbars et de manière particulièrement préférée dans la plage de 1 à 100 mbars.

10. Procédé selon l'une des revendications 1 à 9, **caractérisé en ce que**
i) au moins une partie de l'eau contenue dans la composition (ZP) est déjà contenue dans la composition (ZE), la composition (ZE) présentant une teneur en eau de 0,05 à 1% en poids, de préférence de 0,08 à 0,8% en poids et de manière particulièrement préférée de 0,1 à 0,5% en poids, par rapport au poids total de la composition (ZE), et/ou
ii) le procédé comprend en outre l'ajout d'eau et/ou
iii) au moins une partie de l'eau contenue dans la composition (ZP) est ajoutée conjointement avec ledit au moins un agent réducteur (R).

11. Procédé selon l'une des revendications 1 à 10, **caractérisé en ce que** la composition (ZP) contient 0,10 à 2% en poids et de préférence 0,15 à 1,5% en poids d'eau, par rapport au poids total de la composition (ZP).

12. Procédé selon l'une des revendications 1 à 11, **caractérisé en ce que** le rapport pondéral de l'eau audit au moins un agent réducteur (R) dans la composition (ZP) est de 100:1 à 1:1, de préférence de 50:1 à 2:1 et de manière particulièrement préférée de 30:1 à 4:1.

13. Composition (ZP) préparée selon le procédé selon l'une des revendications 1 à 12.

14. Utilisation de la composition (ZP) selon la revendication 13 pour la préparation de tensioactifs, de médicaments et d'agents phytosanitaires, de stabilisants, d'agents de protection contre la lumière, de polymères, d'isocyanates, de durcisseurs pour résines époxy, de catalyseurs pour polyuréthanes, d'intermédiaires pour la préparation de composés d'ammonium quaternaire, de plastifiants, d'inhibiteurs de corrosion, de résines synthétiques, d'échangeurs d'ions, d'adjuvants textiles, de colorants, d'accélérateurs de vulcanisation, d'émulsifiants et/ou comme substances de départ pour la préparation d'urées et de polyurées.
